# EUROPÄISCHE PATENTSCHRIFT

(11) **EP 1 942 865 B1**
(45) Veröffentlichungstag und Bekanntmachung des Hinweises auf die Patenterteilung: **12.10.2011**
(21) Anmeldenummer: 06829919.7
(22) Anmeldetag: 24.10.2006
(51) Int. Cl.: A61K 8/34, A61K 8/36, A61Q 9/02, A61Q 19/00

(54) **Verwendung von 1,2-Alkandiolen zur Verbesserung des Hautgefühls von alkalischen Reinigungszubereitungen**
Use of 1,2-alkanediols for improving the feeling of alkaline cleansing preparations on skin
Utilisation des 1,2-alcane-diols pour améliorer la sensation sur la peau des préparations de nettoyage alcalines

(30) Priorität: 25.10.2005 DE 102005051868
(43) Veröffentlichungstag der Anmeldung: 16.07.2008
(73) Patentinhaber: Beiersdorf AG, 20253 Hamburg (DE)
(72) Erfinder: RUPPERT, Stephan, 20259 Hamburg (DE); ALBRECHT, Harald, CH-8320 Fehraltorf (CH); KÜTHER, Jörg, 25469 Halstenbek (DE); AECHTNER, Anja, 68165 Mannheim (DE); NIELSEN, Jens, 24558 Henstedt-Ulzburg (DE); BLECKMANN, Andreas, 22926 Ahrensburg (DE); ARGEMBEAUX, Horst, 21465 Wentorf (DE); DETERT, Marion, 22455 Hamburg (DE); THOMPSON, Susan, Chelmsford CM1 2NF (GB); KÖHLER, Manuela, 20144 Hamburg (DE)
(86) Internationale Anmeldenummer: PCT/EP2006/067701
(87) Internationale Veröffentlichungsnummer: WO 2007/048782

(56) Entgegenhaltungen:
- EP-A- 0 336 803
- EP-A- 0 451 949
- WO-A-01/79418
- WO-A-95/01151
- DE-A- 10 217 628
- US-A- 5 308 526
- DATABASE WPI Week 200464 Derwent Publications Ltd., London, GB; AN 2004-656547 XP002422881 & JP 2004 250332 A (POLA CHEM. IND. INC.) 9. September 2004 (2004-09-09)
- DATABASE WPI Week 200544 Derwent Publications Ltd., London, GB; AN 2005-429893 XP002422882 & JP 2005 154353 A (POLA CHEM. IND. INC.) 16. Juni 2005 (2005-06-16)

## Beschreibung

Die vorliegende Erfindung betrifft alkalische Reinigungszubereitungen mit verbessertem Hautgefühl.

Der Wunsch nach einem sauberen und gepflegten Äußeren ist wohl so alt wie die Menschheit. Unreine Haut und ein ungepflegtes Haarkostüm bieten idealen Nährboden und Heimstatt für Krankheitserreger und Parasiten aller Art. Die Lust an der Körperhygiene wurde stetig verstärkt, als in den 60er Jahren des 20 Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Alkalische Reinigungsformulierungen zum Duschen, zum Baden, zur Gesichtsreinigung oder zur Vorbereitung der Haut auf die Rasur sind seit langem bekannt und werden insbesondere im asiatischen Markt bereits eingesetzt. Durch ihre im Vergleich zu herkömmlichen Duschgelen spezielle Tensidzusammensetzung bieten diese Formulierungen dem Verbraucher einen besonders feinporigen Schaum.

Entscheidender Nachteil dieser Produkte ist, dass sie einen sehr hohen pH-Wert aufweisen und deshalb nach dem Waschen ein trockenes Hautgefühl zurücklassen.

In manchen Kulturkreisen wird ein solches trockenes oder auch als stumpf bezeichnetes Hautgefühl als durchaus angenehm und erwünscht angesehen. Dies trifft insbesondere für zahlreiche asiatische Länder mit hoher Luftfeuchtigkeit zu.

In Ländern und Kontinenten mit eher trockenen Klimaten hingegen verbinden Konsumenten mit einer trockenen, sich stumpf anfühlenden Haut deutlich negative Empfindungen und Assziationen. Es ist deshalb ein durchaus anspruchsvolles und lohnendes Ziel die guten Reinigungseigenschaften von seifenhaltigen Produkten mit einem für Konsumenten in diesen Ländern akzeptierten Hautgefühl einer glatten, weichen und gut befeuchteten Haut zu kombinieren.

Es war daher die Aufgabe der vorliegenden Erfindung, die Mängel des Standes der Technik zu beseitigen und alkalische Reinigungszubereitungen mit besonders gutem Hautgefühl zu entwickeln.

Überraschend wurde gefunden, dass der Zusatz von 1,2-Alkandiolen zu den erfindungsgemäßen Reinigungsprodukten insbesondere das Hautgefühl der Zubereitung erheblich verbessert.

Als besonders geeignet hat sich das 1,2-Hexandiol herausgestellt.

Der Einsatz von 1,2-Hexandiol in kosmetischen Zubereitungen ist dem Fachmann an sich bekannt, doch konnte der Stand der Technik nicht den Weg zur vorliegenden Erfindung weisen.

Die US 9,308,526 offenbart feuchtigkeitsspendende Reinigungszusammensetzungen, die hexylene glycol enthalten.

Die WO 95/01151 offenbart die Verwendung von Alkandiolen mit 5-7 Kohlenstoffatomen in kosmetischen Zubereitungen. Erfindungsgemäße Zubereitungen mit 1,2-Hexandiol sind jedoch nicht explizit offenbart.

Die DE 103 41 179 offenbart Deodorantzusammensetzungen mit einer Kombination aus Alkan-1,2-diolen, u. a. 1,2-Octandiol und alpha- und/oder beta-Hydroxysäuren. Erfindungsgemäße Zubereitungen mit 1,2-Hexandiol sind jedoch nicht offenbart.

EP 1078638 offenbart Lichtschutzzubereitungen mit hohen Konzentrationen an 1,2-Hexandiol.

Zwar kennt der Stand der Technik Reinigungsgele enthaltend quervernetzte AcrylatCopolymere. So beschreiben die WO 01/076552, WO 01/019946, EP 1291000, EP 1291001, EP1291002, EP1291003 und EP 1291005 derartige Zubereitungen. Doch konnten diese Schriften nicht den Weg zur vorliegenden Erfindung weisen.

All diesen Schriften fehlt es an Hinweisen, das 1,2-Hexandiol hilfreich verwendet werden kann, um das Hautgefühl von erfindungsgemäßen Zubereitungen während und insbesondere nach dem Abwaschen deutlich zu verbessern.

Daher ergab es sich für den Fachmann überraschend und nicht vorhersehbar, das eine alkalische Reinigungszubereitung enthaltend mehr als 5 Gew.-%, bevorzugt mehr als 15 Gew.-%, bevorzugt mehr als 30 Gew.-%, besonders bevorzugt mehr als 50 Gew.-% Alkalifettsäuresalze mit einer C- Kettenlänge zwischen 10 und 20 Kohlenstoffatomen, ein oder mehrere 1,2-Alkandiole mit 5, 6, 7 oder 8 Kohlenstoffatomen, wobei die Zubereitung einen pH-Wert größer 8 aufweist, den Mängeln des Standes der Technik abhilft und insbesondere eine Verbesserung des Hautgefühls nach Anwendung der Zubereitung ermöglicht. Darüber hinaus wird eine gute Reinigungsleistung erreicht. Die erfindungsgemäße Zubereitung weist darüber hinaus vielfache Vorteile auf:
- Neben den Pflege- und/ oder Reinigungseigenschaften stellt die Handhabung eines kosmetischen Produktes einen für den Verbraucher sehr wichtigen Punkt dar. Die hier beschriebene Formulierung verfügt über sehr gute Anwendungseigenschaften. Sie lässt sich leicht aus Tube, Flasche oder Flasche mit Pumpe entnehmen und dosieren. Zudem ist sie auf der Haut gut verteilbar und lässt sich nach der Anwendung einfach abspülen.
- Das Produkt weist eine sehr gute Langzeitstabilität auf. Diese garantiert über den gesamten Verwendungszeitraum eine gleich bleibende Produktqualität und Produktleistung.
- Herausragend gute Schaum- und sehr gute Reinigungsleistung des Produktes.

In erfindungsgemäßen Zubereitungen ist es von großem Vorteil, wenn das Alkandiol 1,2-Hexandiol ist. Weiterhin ist es von großem Vorteil, wenn mehr als 0,3 Gew.-%, bevorzugt mehr als 0,6 Gew.-%, besonders bevorzugt mehr als 0,8 Gew.-% Alkandiol enthalten sind. Weiterhin ist es von großem Vorteil, wenn weniger als 10 bevorzugt weniger als 5 besonders bevorzugt weniger als 2,5 Gew.-% Alkandiol enthalten sind. Weiterhin ist es von großem Vorteil, wenn der pH Wert der Formulierung größer 9 bevorzugt größer 10 ist. Weiterhin ist es von großem Vorteil, wenn mehr als 1 Gew.-%, bevorzugt mehr als 2 Gew.-% einer Alkalimetallauge oder deren Salze enthalten und / oder zu deren Herstellung verwendet wurden. Weiterhin ist es von großem Vorteil, wenn eine Transmission von 50% bis 100%, besonders bevorzugt von 55 bis 98% gemessen durch Transmissionsmessung bei einer Wellenlänge von 420 nm aufweist. Weiterhin ist es von großem Vorteil, wenn mehr als 0,1 Gew.-%, bevorzugt mehr als 0,5 Gew.-%, besonders bevorzugt mehr als 1 Gew.-% eines natürlichen Öles mit einem Schmelzpunkt größer 20 °C enthält. Weiterhin ist es von großem Vorteil, wenn die Zubereitung mehr als 1,5 Gew.-%, bevorzugt mehr als 3 Gew.-%, besonders bevorzugt mehr als 5 Gew.-% eines synthetischen Tensids enthält. Weiterhin ist es von großem Vorteil, wenn mehr als 0,5 Gew.-%, bevorzugt mehr als 1 Gew.-%, besonders bevorzugt mehr als 2 Gew.-% Glycerin enthält. Weiterhin ist es von großem Vorteil, wenn zusätzlich mindestens ein polymerer Verdicker enthalten ist. Weiterhin ist es von großem Vorteil, wenn die Zubereitung in flüssiger Form vorliegt. Weiterhin ist es von großem Vorteil, wenn die Zubereitung als festes Reinigungsstück vorliegt. Weiterhin umfasst die Erfindung ein Trägermaterial getränkt oder bestreut mit einer erfindungsgemäßen Zubereitung. Weiterhin umfasst die Erfindung die Verwendung einer erfindungsgemäßen Zubereitung als Rasiergel, Rasiervorbereitungszubereitung oder zur Körperreinigung. Weiterhin umfasst die Erfindung die Verwendung von 1,2-Alkandiolen mit 5,6,7 oder 8 Kohlenstoffatomen zur Verbesserung des Hautgefühls von alkalischen Reinigungszubereitungen.

Erfindungsgemäß bevorzugte Ausführungsformen der vorliegenden Erfindung sind dadurch gekennzeichnet, dass die Zubereitung einen oder mehrere Konservierungsstoffe in einer Konzentration von 0,1 bis 2,0 Gewi.-% und besonders bevorzugt von 0,2 bis 1,2 Gew.-%, jeweils bezogen auf das Gesamtgewicht der Zubereitung, enthalten kann.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenen Konservierungsmittel vorteilhaft im Sinne der vorliegenden Erfindung.

Erfindungsgemäß besonders bevorzugt ist dabei der Einsatz von Parabenen und Phenoxyethanol als Konservierungsmittel.

Vorteilhaft im Sinne der vorliegenden Erfindung ist es, wenn das erfindungsgemäße Reinigungsgel einen oder mehrere Komplexbildner in einer Gesamtkonzentration von 0,1 bis 2,0 Gew.-% und besonders bevorzugt in einer Konzentration von 0,2 bis 1,0 Gew.-%, jeweils bezogen auf das Gesamtgewicht der Zubereitung, enthält.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA), Tetrasodium Iminodisuccinate (IDS), und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Erfindungsgemäß besonders bevorzugt ist es Hydroxyethylendiaminotriessigsäure und insbesondere das Natriumsalz der Hydroxyethylendiaminotriessigsäure (Na₃HEDTA) als Komplexbildner einzusetzen.

Es ist erfindungsgemäß vorteilhaft, wenn das erfindungsgemäße Reinigungsggel einen oder mehrere Hautbefeuchtungsmittel in einer Konzentration von 0,5 bis 10 Gew.-% und bevorzugt in einer Konzentration von 2,0 bis 6,0 Gew.-%, jeweils bezogen auf das Gesamtgewicht der Zubereitung, enthält.

Erfindungsgemäß vorteilhafte Hautbefeuchtungsmittel sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Erfindungsgemäß besonders bevorzugt ist dabei der Einsatz von Glycerin als Hautbefeuchtungsmittel.

Besonders interessant im Sinne der Anmeldung sind transparente Zubereitungen, wobei der Grad der Transmission 50% bis 100%, besonders bevorzugt von 55 bis 98% gemessen bei 420 nm betragen sollte.

Eine Transmissionsmessung kann mit einem handelsübliches Photometer (Typ Hewlett Packard 84521 Dioden Array Spectrophotometer. Das Seifengel wird zuerst 1:1000 mit destilliertem Wasser (w/w) verdünnt, anschließend wird es auf einem Schüttler so lange geschüttelt, bis eine homogene Lösung entstanden ist. Die Messung erfolgt im sichtbaren Bereich (400 - 800 m) bei 20°C .in Einweg-Kunststoffküvetten gegen destilliertes Wasser als Blindwert.

Es ist erfindungsgemäß vorteilhaft, wenn das erfindungsgemäße Reinigungsgel Abrasiva (z. B. Peelingpartikel aus Polyethylen), Glitterstoffe, Effektstoffe, Farbschlieren, Gasblasen (insbesondere Luftblasen), Perlglanzpigmente, Glimmer, Öl- und/oder Emulsionströpchen enthält.

Auch ist es vorteilhaft im Sinne der vorliegenden Erfindung, wenn das erfindungsgemäße Reinigungsgel oder Reinigungsstück einen oder mehrere Farbstoffe enthält. Dabei können insbesondere alle bekannten, wasserlöslichen und für die Kosmetik zugelassenen Farbstoffe der Zubereitung zugesetzt werden.

Selbstverständlich ist es erfindungsgemäß von Vorteil, dem erfindungsgemäßen Reinigungsgel oder Reinigungsstück weitere kosmetische Wirk-, Hilfs- und Zusatzstoffe zuzusetzen. Die Nachfolgende Aufzählung gibt eine kleine Auswahl erfindungsgemäß vorteilhafter weiterer Zusätze, die aber keinesfalls die vorliegende Erfindung auf diese Verbindungen beschränken soll.

Die wässrige Phase der erfindungsgemäßen Reinigungsgele kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Perlglanzagentien, Antischuppenwirkstoffe, Pflanzenextrakte, Vitamine, Wirkstoffe.

Besonders vorteilhafte Zubereitungen werden ferner erhalten, wenn als Zusatz- oder Wirkstoffe Antioxidantien eingesetzt werden.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder ß-Alanin sowie 8-Hexadecen-1,16-dicarbonsäure (Dioic acid, CAS-Nummer 20701-68-2; vorläufige INCI-Bezeichnung Octadecendioic acid), Licochalcon A, Ascorbinsäure und deren Derivate Vitamin E und dessen Derivate, Vitamin A und dessen Derivate,γ-Oryzanol, Panthenol und/oder Niacinamid.

Ein weiterer erfindungsgemäß vorteilhafter Wirkstoff stellt beispielsweise Polidocanol dar.

Die Menge der Wirkstoffe (eine oder mehrere Verbindungen) in den Zubereitungen beträgt erfindungsgemäß vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Erfindungsgemäß vorteilhaft wird die erfindungsgemäße Zubereitung in einer Tube, einem Pumpspender oder einer Flasche aufbewahrt und aus dieser heraus angewendet. Derartige Verpackungsbehältnisse sind erfindungsgemäß bevorzugt transparent oder transluzent.

Daher sind auch Tuben, Pumpspender oder Flaschen enthaltend eine erfindungsgemäße Zubereitung, erfindungsgemäß, wobei transparente oder transluzente Verpackungsbehältnisse erfindungsgemäß bevorzugt sind.

Vorteilhaft im Sinne der vorliegenden Erfindung ist es ferner, das erfindungsgemäße Reinigungsgel oder Reinigungspulver auf ein Trägermaterial (z.B. ein Tuch, Pad, Wattebausch) aufzutragen. Derartige Träger bestehen vorzugsweise aus Viskose-, Baumwolle- und/oder Polyesterfasern.

Erfindungsgemäß ist daher auch ein Trägermaterial (z.B. ein Tuch, Pad, Wattebausch) getränkt mit einem erfindungsgemäßen Reinigungsgel, wobei das Trägermaterial bevorzugt aus Viskose-, Baumwolle- und/oder Polyesterfasern aufgebaut ist.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

### Beispiele:

Flüssige alkalische Reinigungsformulierungen:

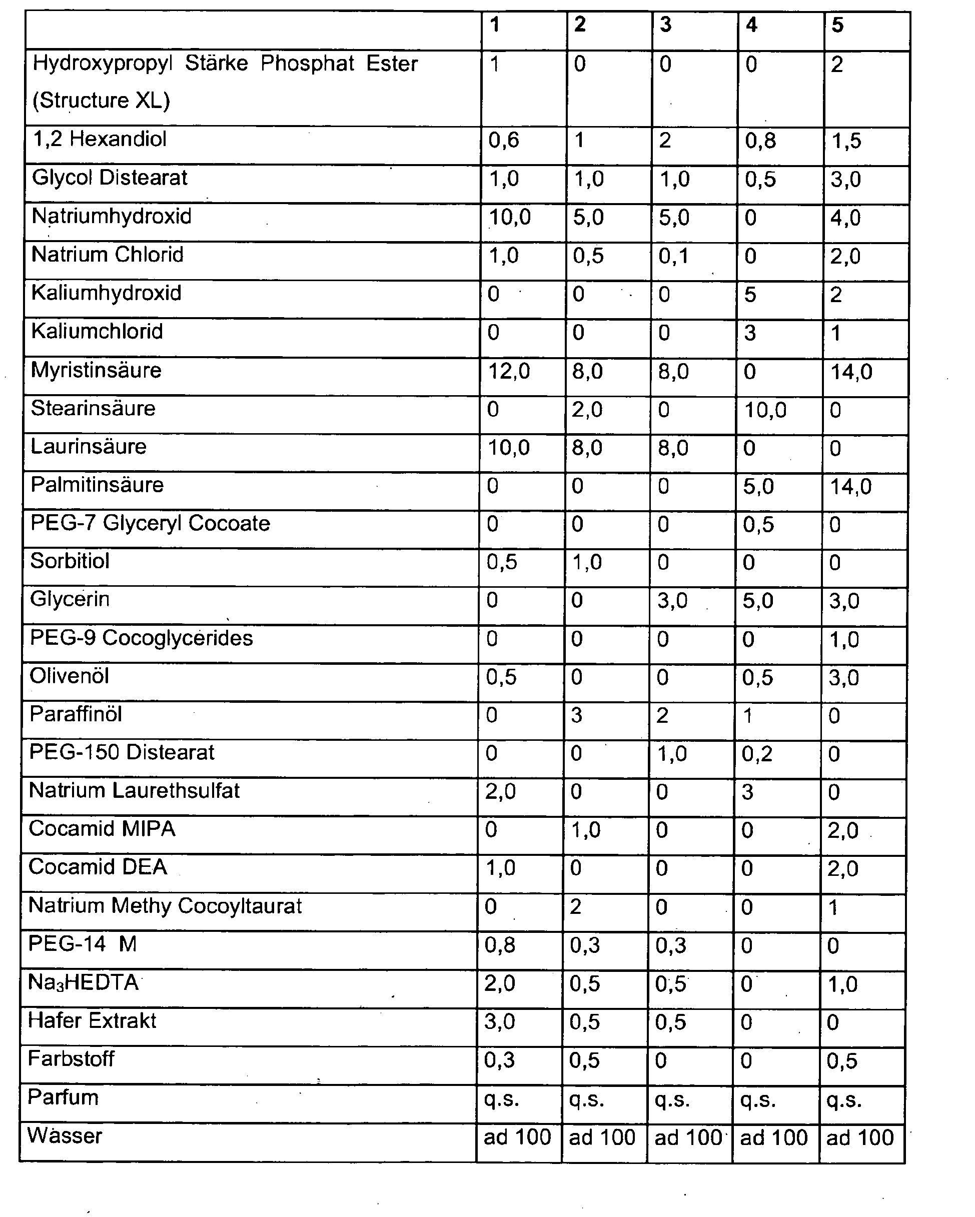

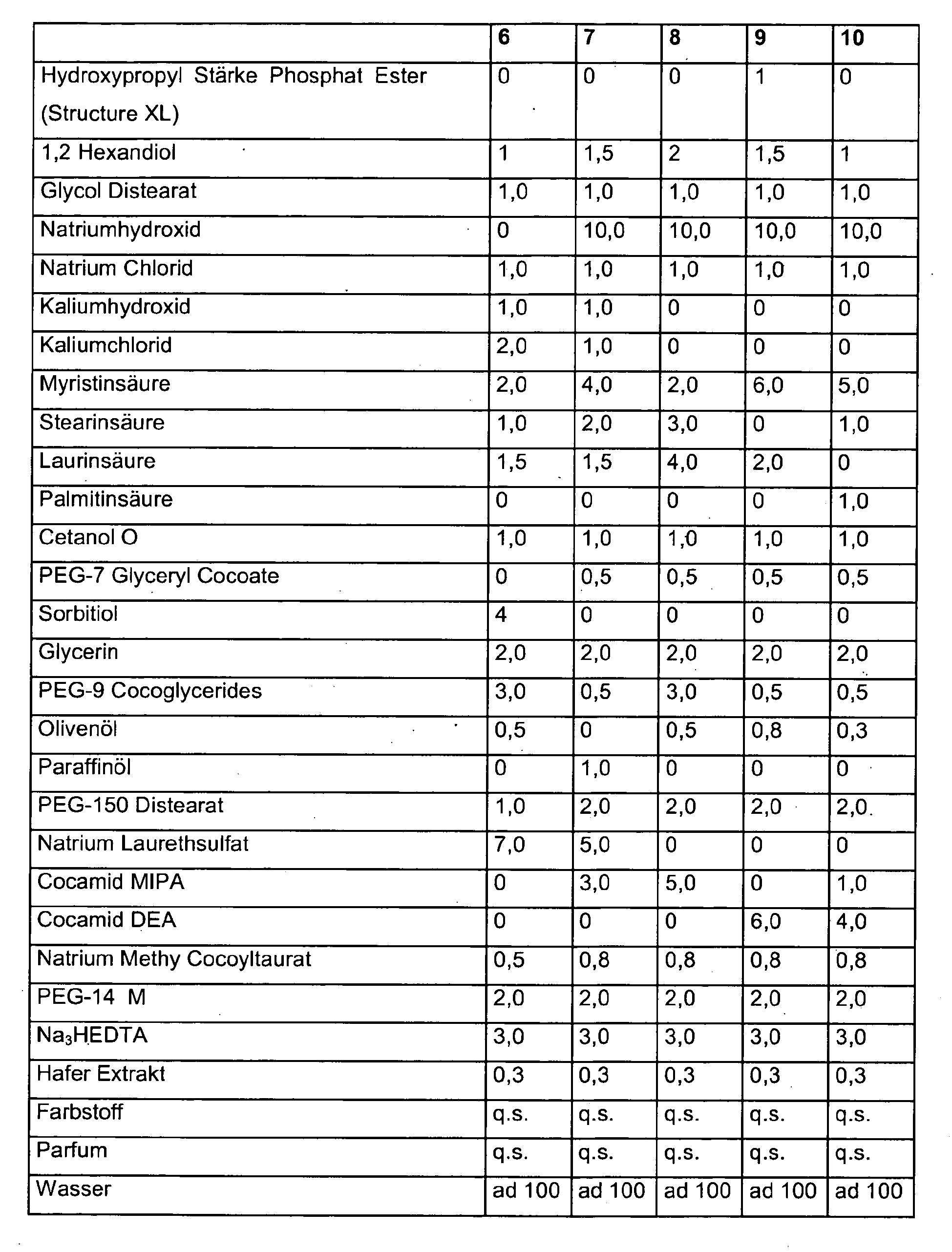

### Feste alkalische Reinigungsformulierungen:

### Seifenherstellung:

Die Grundseifennudeln werden mit dem Farbslurry und den übrigen Komponenten in einen üblichen Seifenmischer (Schneckenmischer mit Lochsieb) dosiert, durch mehrmaliges Vermischen homogenisiert, über eine Strangpresse ausgetragen, geschnitten und in üblicher Weise zu Stücken verarbeitet.

### Grundseifen:

| | Gew.-% | Gew.-% | Gew.-% | Gew.-% | Gew.-% |
|---|---|---|---|---|---|
| Natriumtallowat | 68,0 | 68,0 | 65,0 | 0 | 68,0 |
| Natriumcocoat | 0 | 17,0 | 17,0 | 82,0 | 17,0 |
| Natriumpalmkernöl | 17,0 | 0 | 0 | 0 | 0 |
| Aqua | 11,0 | 11,0 | 12,0 | 12,0 | 11,0 |
| NaCl | 0,5 | 0,5 | 0,5 | 0,5 | 0,5 |
| EDTA | 0,2 | 0 | 0,2 | 0,2 | 0,2 |
| Natriumetidronat | 0,1 | 0,1 | 0 | 0,1 | 0,1 |
| Glycerin | 2,5 | 2,5 | 0,5 | 2,5 | 2,5 |
| Natrium-Palmkernfettsäuresalze | ad 100,0 | ad 100,0 | ad 100,0 | ad 100,0 | ad 100,0 |

### Farbslurry:

| | Gew.-% | Gew.-% | Gew.-% |
|---|---|---|---|
| Wollwachsalkohol | 2,0 | 15,0 | 0 |
| Paraffinum Liquidum | 33,0 | 45,0 | 33,0 |
| Prunus Dulcis | 45,0 | 0 | 30,0 |
| Disteardimoniumhectorit | 1,0 | 3,0 | 1,0 |
| Farbstoff | 0 | 1,5 | 0,5 |
| TiO₂ | 13,0 | 13,0 | 25,0 |
| Wasser | ad 100,0 | ad 100,0 | ad 100,0 |

### Seifenstücke:

| | Gew.-% | Gew.-% | Gew.-% | Gew.-% |
|---|---|---|---|---|
| 1,2 Hexandiol | 0,8 | 1 | 1 | 1,5 |
| Hydroxypropyl Stärke Phosphat Ester (Structure XL) | 1,0 | 0 | 0 | 0 |
| Farbslurry | 3,0 | 3,0 | 3,0 | 3,0 |
| Grundseife | 85,0 | 86,8 | 85,9 | 91,0 |
| Paraffin | 1,5 | 1,0 | 1,0 | 2,0 |
| Polyethylenglycol-150 | 0 | 0 | 0 | 2,0 |
| Talkum | 7,0 | 9,0 | 0 | 6,0 |
| Parfum | 1,0 | 1,5 | 1,0 | 2,0 |
| Na₂S₂O₃ | 0,4 | 0,5 | 0,4 | 0,7 |
| Octyldodecanol | 0,5 | 0,5 | 0,5 | 0,5 |
| Wasser | ad 100,0 | ad 100,0 | ad 100,00 | ad 100,0 |

### Combibar:

| | Gew.-% | Gew.-% | Gew.-% | Gew.-% |
|---|---|---|---|---|
| 1,2 Hexandiol | 0,8 | 2 | 1 | 1,5 |
| Hydroxypropyl Stärke Phosphat Ester (Structure XL) | 0 | 1 | 0 | 0 |
| Natrium Cocoyl Isetionat | 30,0 | 40,0 | 37,0 | 43,5 |
| Stearinsäure | 22,7 | 22,7 | 22,7 | 22,7 |
| Grundseife | 23 | 15 | 23 | 18 |
| Dinatrium Lauryl Sulfosuccinat | 17 | 9,5 | 9,5 | 12 |
| Talkum | 0 | 5,0 | 5,0 | 5,0 |
| Kokusnussfett-Säure | 3,5 | 3,3 | 3,3 | 3,8 |
| PEG-150 | 2,0 | 2,0 | 2,0 | 2,0 |
| Paraffin | 1,0 | 2,0 | 2,0 | 3,0 |
| TiO₂ | 0,5 | 0,5 | 0,5 | 0 |
| Kalium Hydroxid | 0,5 | 1,0 | | |
| Panthenol | 0,5 | 0,3 | 1,0 | 0 |
| Lanolin Alkohol | 0,1 | 0,1 | 0 | 0,1 |
| Parfüm | 1,0 | 1,5 | 2,0 | 1,5 |
| Wasser | ad 100,0 | ad 100,0 | ad 100,00 | ad 100,0 |

### Syndet:

| | **1** | **2** | **3** | **4** | **5** |
|---|---|---|---|---|---|
| 1,2 Hexandiol | 1 | 1,5 | 1 | 1,5 | 2 |
| Hydroxypropyl Stärke Phosphat Ester (Structure XL) | 14.0 | 28.0 | 20.0 | 20.0 | 11.0 |
| Dinatrium Lauryl Sulfosuccinate | 20.0 | 15.0 | 21.0 | 15.0 | 34.0 |
| Natrium Cocoyl Isethionate | 12.8 | 12.8 | 12.8 | 12.8 | 12.8 |
| Cetearyl Alkohol | 12.0 | 12.0 | 12.0 | 12.0 | 12.0 |
| Glyceryl Stearate | 11.8 | 11.8 | 11.8 | 14.0 | 11.8 |
| Paraffin | 4.5 | 11.5 | 11.5 | 7.5 | 4.5 |
| Weizenstärke | 2.0 | 2.0 | 2.0 | 2.0 | 2.0 |
| Cocamidopropyl Betaine | 1.5 | 1.5 | 1.5 | 2,0 | 1.5 |
| Zitronensäure | 2.0 | 1.0 | 1.0 | 2.0 | 1.0 |
| PEG-150 | 3,0 | 0.0 | 0.5 | 0.5 | 3,0 |
| Octyldodecanol | 0 | 1,0 | 0 | 0,5 | 0 |
| Jojobaöl | 3 | 0 | 0 | 0 | 0 |
| Kalium Hydroxid | 1 | 0,5 | 0 | 0,5 | 0 |
| Talkum | 0,5 | 1,0 | 0 | 0 | 0 |
| Farbstoff | 0.0 | 0.5 | 0.5 | 0.5 | 0.5 |
| TiO₂ | 0.1 | 0.1 | 0.1 | 0.5 | 0.1 |
| Diammoniumcitrat | 0.1 | 0.1 | 0.1 | 0.1 | 0.0 |
| Lanolin Alkohol | 0,1 | 0,1 | 0 | 0,1 | 0,1 |
| Parfum | q.s. | q.s. | q.s. | q.s. | q.s. |
| Wasser | ad 100 | ad 100 | ad 100 | ad 100 | ad 100 |

### Rasierschaum

| | **1** | **2** | **3** | **4** | **5** | **6** |
|---|---|---|---|---|---|---|
| Stearinsäure | 6,20 | 6,20 | 5,20 | 6,00 | 7,50 | 6,00 |
| Myristinsäure | --- | --- | --- | 1,00 | --- | 1,00 |
| Palmitinsäure | --- | --- | 1,00 | --- | --- | --- |
| Polyethylenglykol(29)cetylether | 2,50 | 2,50 | 2,50 | 2,00 | 0,50 | 2,00 |
| Stearylalkohol | 0,50 | 0,50 | 0,50 | --- | --- | --- |
| Cetylalkohol | --- | --- | --- | --- | 0,50 | --- |
| Glycerin | 2,40 | 2,40 | 1,20 | 3,00 | 3,00 | 5,00 |
| Sorbitol | --- | --- | 1,20 | 2,00 | --- | --- |
| Triethanolamine | 3,00 | 3,00 | 3,00 | --- | 3,50 | 4,00 |
| Kalium Hydroxid | --- | --- | --- | 1,50 | --- | --- |
| Propylparaben | 0,08 | 0,08 | 0,40 | 0,08 | 0,08 | 0,08 |
| Methylparaben | 0,12 | 0,12 | 0,40 | 0,12 | 0,12 | 0,12 |
| 1,2-Hexandiol | 0,80 | 2,00 | 0,60 | 1,00 | 1,50 | 1,50 |
| Parfum | q.s. | q.s. | q.s. | q.s. | q.s. | q.s. |
| Wasser | ad 100 | ad 100 | ad 100 | ad 100 | ad 100 | ad 100 |

### Rasiergel

| | **1** | **2** | **3** | **4** | **5** | **6** |
|---|---|---|---|---|---|---|
| Stearinsäure | --- | 2,00 | --- | 6,00 | 6,00 | 1,50 |
| Palmitinsäure | 8,50 | 6,50 | 8,50 | --- | --- | 7,00 |
| Myristinsäure | --- | --- | --- | 2,00 | 2,00 | --- |
| Polyethylenglykol(25)cetearlylether | 6.00 | 6,00 | 6,00 | 2,00 | --- | 2,00 |
| Glycerin | --- | 1,00 | --- | --- | ---- | --- |
| Sorbitol | 3,30 | 2,30 | 3,30 | 5,50 | 7,00 | 5,50 |
| Hydroxyethylcellulose | 0,50 | 0,50 | 0,50 | 0,50 | 0,50 | 0,30 |
| Mineral Öl | 1,00 | 1,00 | 1,00 | --- | --- | --- |
| Triethanolamine | 6,70 | 6,70 | 6,70 | --- | 4,20 | 5,00 |
| Kalium Hydroxid | --- | --- | --- | 1,60 | --- | --- |
| Propylparaben | 0,24 | 0,08 | 0,24 | 0,24 | 0,24 | 0,24 |
| Methylparaben | 0,36 | 0,12 | 0,36 | 0,36 | 0,36 | 0,36 |
| 1,2-Hexandiol | 0,80 | 2,00 | 0,60 | 1,00 | 1,50 | 1,50 |
| Parfum | q.s. | q.s | q.s. | q.s | q.s | q.s |
| Wasser | ad 100 | ad 100 | ad 100 | ad 100 | ad 100 | ad 100 |

## Patentansprüche

1. Alkalische Reinigungszubereitung enthaltend mehr als 5 Gew.-%, bevorzugt mehr als 15 Gew.-%, bevorzugt mehr als 30 Gew.-%, besonders bevorzugt mehr als 50 Gew.-% Alkalifettsäuresalze mit einer C- Kettenlänge zwischen 10 und 20 Kohlenstoffatomen, ein oder mehrere 1,2-Alkandiole mit 5,6 oder 7 Kohlenstoffatomen, wobei die Zubereitung einen pH-Wert größer 8 aufweist, **dadurch gekennzeichnet, dass** weniger als 10 bevorzugt weniger als 5 besonders bevorzugt weniger als 2,5 Gew.-% Alkandiol enthalten sind.

2. Zubereitung nach Patentanspruch 1 **dadurch gekennzeichnet, dass** das Alkandiol 1,2-Hexandiol ist.

3. Zubereitung nach einem der vorhergehenden Ansprüche **dadurch gekennzeichnet, dass** mehr als 0,3 Gew.-%, bevorzugt mehr als 0,6 Gew.-%, besonders bevorzugt mehr als 0,8 Gew.-% Alkandiol enthalten sind.

4. Zubereitung nach einem der vorhergehenden Ansprüche **dadurch gekennzeichnet, dass** der pH-Wert der Formulierung größer 9, bevorzugt größer 10 ist.

5. Zubereitung nach einem der vorhergehenden Ansprüche **dadurch gekennzeichnet, dass** sie mehr als 1 Gew.-%, bevorzugt mehr als 2 Gew.-% einer Alkalimetallauge oder deren Salze enthalten und / oder zu deren Herstellung verwendet wurden.

6. Zubereitung nach einem der vorhergehenden Ansprüche **dadurch gekennzeichnet, dass** sie eine Transmission von 50% bis 100%, besonders bevorzugt von 55 bis 98% gemessen durch Transmissionsmessung bei einer Wellenlänge von 420 nm aufweist.

7. Zubereitung nach einem der vorhergehenden Ansprüche **dadurch gekennzeichnet, dass** sie mehr als 0,1 Gew.-%, bevorzugt mehr als 0,5 Gew.-%, besonders bevorzugt mehr als 1 Gew.-% eines natürlichen Öles mit einem Schmelzpunkt größer 20 °C enthält.

8. Zubereitung nach einem der vorhergehenden Ansprüche **dadurch gekennzeichnet, dass** sie mehr als 1,5 Gew.-%, bevorzugt mehr als 3 Gew.-%, besonders bevorzugt mehr als 5 Gew.-% eines synthetischen Tensids enthält.

9. Zubereitung nach einem der vorhergehenden Ansprüche **dadurch gekennzeichnet, dass** die Zubereitung mehr als 0,5 Gew.-%, bevorzugt mehr als 1 Gew.-%, besonders bevorzugt mehr als 2 Gew.-% Glycerin enthält.

10. Zubereitung nach einem der vorangehenden Ansprüche **dadurch gekennzeichnet, dass** zusätzlich mindestens ein polymerer Verdicker enthalten ist.

11. Zubereitung nach einem der vorhergehenden Ansprüche **dadurch gekennzeichnet, dass** sie in flüssiger Form vorliegt.

12. Zubereitung nach einem der vorhergehenden Ansprüche **dadurch gekennzeichnet, dass** sie als festes Reinigungsstück vorliegt.

13. Trägermaterial getränkt oder bestreut mit einer Zubereitung nach einem der vorhergehenden Ansprüche.

14. Verwendung einer Zubereitung nach einem der vorangehenden Ansprüche als Rasiergel, Rasiervorbereitungszubereitung oder zur Körperreinigung.

15. Verwendung von 1,2-Alkandiolen mit 5, 6 oder Kohlenstoffatomen zur Verbesserung des Hautgefühls von alkalischen Reinigungszubereitungen.

## Claims

1. Alkaline cleansing preparation comprising more than 5% by weight, preferably more than 15% by weight, preferably more than 30% by weight, particularly preferably more than 50% by weight, of alkali metal fatty acid salts with a carbon chain length between 10 and 20 carbon atoms, one or more 1,2-alkanediols having 5, 6 or 7 carbon atoms, where the preparation has a pH greater than 8, **characterized in that** less than 10% by weight, preferably less than 5% by weight, particularly preferably less than 2.5% by weight, of alkanediol are present.

2. Preparation according to Claim 1, **characterized in that** the alkanediol is 1,2-hexanediol.

3. Preparation according to one of the preceding claims, **characterized in that** more than 0.3% by weight, preferably more than 0.6% by weight, particularly preferably more than 0.8% by weight, of alkanediol are present.

4. Preparation according to one of the preceding claims, **characterized in that** the pH of the formulation is greater than 9, preferably greater than 10.

5. Preparation according to one of the preceding claims, **characterized in that** more than 1% by weight, preferably more than 2% by weight, of an alkali metal hydroxide solution or salts thereof are present and/or have been used for its preparation.

6. Preparation according to one of the preceding claims, **characterized in that** it has a transmission of from 50% to 100%, particularly preferably of from 55 to 98%, measured by transmission measurement at a wavelength of 420 nm.

7. Preparation according to one of the preceding claims, **characterized in that** it comprises more than 0.1% by weight, preferably more than 0.5% by weight, particularly preferably more than 1% by weight, of a natural oil with a melting point greater than 20°C.

8. Preparation according to one of the preceding claims, **characterized in that** it comprises more than 1.5% by weight, preferably more than 3% by weight, particularly preferably more than 5% by weight, of a synthetic surfactant.

9. Preparation according to one of the preceding claims, **characterized in that** the preparation comprises more than 0.5% by weight, preferably more than 1% by weight, particularly preferably more than 2% by weight, of glycerol.

10. Preparation according to one of the preceding claims, **characterized in that** additionally at least one polymeric thickener is present.

11. Preparation according to one of the preceding claims, **characterized in that** it is present in liquid form.

12. Preparation according to one of the preceding claims, **characterized in that** it is present as a solid cleansing bar.

13. Carrier material impregnated or sprinkled with a preparation according to one of the preceding claims.

14. Use of a preparation according to one of the preceding claims as shaving gel, pre-shave preparation or for cleansing the body.

15. Use of 1,2-alkanediols having 5, 6 or 7 carbon atoms for improving the feeling of alkaline cleansing preparations on skin.

## Revendications

1. Préparation alcaline de nettoyage, contenant plus de 5 % en poids, de préférence plus de 15 % en poids, de préférence plus de 30 % en poids, de façon particulièrement préférée plus de 50 % en poids de sels alcalins d'acides gras ayant une longueur de chaîne comprise entre 10 et 20 atomes de carbone, un ou plusieurs 1,2-alcanediols ayant 5, 6 ou 7 atomes de carbone, la préparation présentant un pH supérieur à 8, **caractérisée en ce que** moins de 10, de préférence moins de 5, de façon particulièrement préférée moins de 2,5 % en poids d'alcanediol sont contenus.

2. Préparation selon la revendication 1, **caractérisée en ce que** l'alcanediol est le 1,2-hexanediol.

3. Préparation selon l'une quelconque des revendications précédentes, **caractérisée en ce que** plus de 0,3 % en poids, de préférence plus de 0,6 % en poids, de façon particulièrement préférée plus de 0,8 % en poids d'alcanediol sont contenus.

4. Préparation selon l'une quelconque des revendications précédentes, **caractérisée en ce que** le pH de la composition est supérieur à 9, de préférence supérieur à 10.

5. Préparation selon l'une quelconque des revendications précédentes, **caractérisée en ce que** plus de 1 % en poids, de préférence plus de 2 % en poids d'une solution de métal alcalin ou de ses sels sont contenus et/ou ont été utilisés pour sa préparation.

6. Préparation selon l'une quelconque des revendications précédentes, **caractérisée en ce qu'**elle présente une transmission de 50 % à 100 %, de façon particulièrement préférée de 55 à 98 %, mesurée par mesure de la transmission à une longueur d'onde de 420 nm.

7. Préparation selon l'une quelconque des revendications précédentes, **caractérisée en ce qu'**elle contient plus de 0,1 % en poids, de préférence plus de 0,5 % en poids, de façon particulièrement préférée plus de 1 % en poids d'une huile naturelle ayant un point de fusion supérieur à 20 °C.

8. Préparation selon l'une quelconque des revendications précédentes, **caractérisée en ce qu'**elle contient plus de 1,5 % en poids, de préférence plus de 3 % en poids, de façon particulièrement préférée plus de 5 % en poids d'un tensioactif synthétique.

9. Préparation selon l'une quelconque des revendications précédentes, **caractérisée en ce que** la préparation contient plus de 0,5 % en poids, de préférence plus de 1 % en poids, de façon particulièrement préférée plus de 2 % en poids de glycérol.

10. Préparation selon l'une quelconque des revendications précédentes, **caractérisée en ce qu'**en outre au moins un épaississant polymère est contenu.

11. Préparation selon l'une quelconque des revendications précédentes, **caractérisée en ce qu'**elle se trouve sous forme liquide.

12. Préparation selon l'une quelconque des revendications précédentes, **caractérisée en ce qu'**elle se trouve sous forme de morceau solide de nettoyage.

13. Matière de support imprégnée ou couverte d'une préparation selon l'une quelconque des revendications précédentes.

14. Utilisation d'une préparation selon l'une quelconque des revendications précédentes, en tant que gel de rasage, préparation de prérasage ou pour le nettoyage du corps.

15. Utilisation de 1,2-alcanediols ayant 5, 6 ou 7 atomes de carbone, pour l'amélioration de la sensation sur la peau de préparations alcalines de nettoyage.
